(19) Europäisches Patentamt
European Patent Office
Office européen des brevets

(11) **EP 3 614 388 B1**

(12) **EUROPEAN PATENT SPECIFICATION**

(45) Date of publication and mention
of the grant of the patent:
**16.10.2024 Bulletin 2024/42**

(51) International Patent Classification (IPC):
**G16B 15/30** (2019.01)

(52) Cooperative Patent Classification (CPC):
**G16B 15/30**

(21) Application number: **19185212.8**

(22) Date of filing: **09.07.2019**

(54) **METHOD AND DEVICE FOR CALCULATING BINDING FREE ENERGY, AND PROGRAM**

VERFAHREN UND VORRICHTUNG ZUR BERECHNUNG DER FREIEN BINDUNGSENERGIE UND PROGRAMM

PROCÉDÉ ET DISPOSITIF DE CALCUL DE L'ÉNERGIE SANS LIAISON ET PROGRAMME

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **20.08.2018 JP 2018154175**

(43) Date of publication of application:
**26.02.2020 Bulletin 2020/09**

(73) Proprietor: **FUJITSU LIMITED**
**Kanagawa, 211-8588 (JP)**

(72) Inventor: **TANIDA, Yoshiaki**
**Kawasaki-shi, Kanagawa 211-8588 (JP)**

(74) Representative: **Haseltine Lake Kempner LLP**
**Cheapside House**
**138 Cheapside**
**London EC2V 6BJ (GB)**

(56) References cited:
EP-A1- 3 327 604      WO-A1-2017/199279
JP-A- 2017 091 180      US-A1- 2009 326 878

- **EMILE APOL ET AL: "Gromacs User Manual version 4.5.4", HTTP://WWW.GROMACS.ORG/, 1 January 2010 (2010-01-01), XP055580924, Retrieved from the Internet <URL:ftp://ftp.gromacs.org/pub/manual/manual-4.5.4.pdf> [retrieved on 20190415]**
- **LIMONGELLI V. ET AL: "Funnel metadynamics as accurate binding free-energy method", PROCEEDINGS OF THE NATIONAL ACADEMY OF SCIENCES, vol. 110, no. 16, 7 March 2013 (2013-03-07), pages 6358 - 6363, XP055892799, ISSN: 0027-8424, Retrieved from the Internet <URL:https://www.pnas.org/content/pnas/110/16/6358.full.pdf> [retrieved on 20220216], DOI: 10.1073/pnas.1303186110**
- **B. R. BROOKS ET AL: "CHARMM: The biomolecular simulation program : CHARMM: The Biomolecular Simulation Program", JOURNAL OF COMPUTATIONAL CHEMISTRY., vol. 30, no. 10, 14 May 2009 (2009-05-14), GB, pages 1545 - 1614, XP055715280, ISSN: 0192-8651, DOI: 10.1002/jcc.21287**

**Description**

FIELD

The embodiments discussed herein relate to a method and device for calculating binding free energy, and a program.

BACKGROUND

When a calculation for estimating binding free energy of a binding structure (composite structure) between a target molecule (e.g., a target protein) and a binding calculation target molecule (e.g., a drug candidate molecule) is executed according to a computer experiment, it has been known that a calculation method using an alchemical thermodynamic cycle is the most accurate (see, for example, John D. Chodera et. al., Alchemical free energy methods for drug discovery: Progress and challenges, Curr Opin Struct Biol. 2011 April; 21(2): 150-160).

However, this method cannot perform quantitative estimation when there are a plurality of stable binding structures (binding poses), hence there are cases that accuracy of calculated binding free energy may be low.

Patent document EP 3327604 A1 relates to a method for calculating binding free energy, where the method includes a plurality of steps each including adding a distance restraint potential between a binding calculation target molecule and a target molecule, wherein the method is a method for calculating binding free energy between the binding calculation target molecule and the target molecule using a computer, and wherein anchor points of the binding calculation target molecule in the plurality of the steps are identical anchor points, and anchor points of the target molecule in the plurality of the steps are different anchor points.

Patent document JP 2017 091180 A relates to a pretreatment method for binding free energy calculation between a target molecule and a drug candidate molecule, an apparatus for performing the pretreatment method, a program for executing the pretreatment method, and a calculation method for binding free energy.

Patent document WO 2017/199279 A1 relates to relates to a method of calculating binding free energy between a target molecule and a binding calculation target molecule, a calculation device, and a program for executing the calculation method.

SUMMARY

It is desirable to provide a method and device for calculating binding free energy and a program thereof, all of which can perform a calculation of binding free energy with high accuracy even when there are a plurality of stable binding structures. The invention is defined by the appended claims.

According to an embodiment of one aspect of the present disclosure, a method for calculating binding free energy is a method for calculating binding free energy between a first substance and a second substance using a computer.

An embodiment may perform a calculation of binding free energy with high accuracy even when there are a plurality of stable binding structures.

BRIEF DESCRIPTION OF DRAWINGS

The invention is described, by way of example only, with reference to the following drawings, in which:

FIG. 1 is a conceptual view illustrating one example of the alchemical path calculation method.
FIG. 2 is a diagram illustrating one example of a free-energy surface.
FIG. 3 is a diagram illustrating another example of a free-energy surface.
FIG. 4A is a diagram illustrating one binding pose among a plurality of binding poses.
FIG. 4B is a diagram illustrating another binding pose among a plurality of binding poses.
FIG. 4C is a diagram illustrating yet another binding pose among a plurality of binding poses.
FIG. 5 is a diagram illustrating a relationship between the harmonic potential having the spring constant $K_\xi$ and another example of the free-energy surface.
FIG. 6 is a diagram illustrating a method for selecting the potential energy data.
FIG. 7 is a flowchart illustrating one example of the disclosed method for calculating binding free energy.
FIG. 8 is a view illustrating a structural example of the disclosed device for calculating binding free energy.
FIG. 9 is a view illustrating another structural example of the disclosed device for calculating binding free energy.
FIG. 10 is a view illustrating another structural example of the disclosed device for calculating binding free energy.
FIG. 11 is a schematic view describing the variable of the harmonic potential of Example 1.

DESCRIPTION OF EMBODIMENTS

Drug discovery refers to a process for designing pharmaceutical products. For example, the drug discovery is performed in the following order.

  (1) Determination of a target molecule
  (2) Searching a lead compound etc.
  (3) Examination of physiological effects
  (4) Safety/toxicity test

It is important in the search of a lead compound etc. (a lead compound and a compound derived from the lead compound) that interaction between each of numerous drug candidate molecules and a target molecule is highly accurately evaluated.

A process for designing pharmaceutical products using a computer may be referred to as IT drug discovery. The technology of the IT drug discovery can be used for drug discovery in general. Among them, use of the IT drug discovery in a search of a lead compound etc. is effective for reducing a time period for and increasing a probability of developing a new drug.

For example, the disclosed technology can be used for a search of a lead compound etc. that is expected to have high pharmacological activity.

(Method for calculating binding free energy)

The disclosed method for calculating binding free energy is performed using a computer.

The method for calculating binding free energy is a method for calculating binding free energy between a first substance and a second substance.

The method for calculating binding free energy includes at least a potential energy determination step, a potential energy selection step, and a binding free energy calculation step, and may further include other steps according to the necessity.

The potential energy determination step includes adding predetermined restraint potential between the first substance and the second substance to determine potential energy of a binding structure between the first substance and the second substance.

The potential energy selection step includes selecting potential energy used for a calculation of binding free energy from potential energy data present within a restraining space corresponding to a state where the maximum level of the predetermined restraint potential is added among the entire potential energy data determined in the potential energy determination step.

The binding free energy calculation step includes calculating binding free energy between the first substance and the second substance using the selected potential energy.

The inventors of the disclosed technology have studied a cause for reducing accuracy of calculations when binding free energy between a binding calculation target molecule and a target molecule is calculated utilizing addition of restraint potential.

When "de novo" drug design is performed using molecular simulation, it is important to highly accurately estimate binding activities (binding free energy) between a drug candidate molecule and target protein. As a method for such the estimation, it has been widely known that the alchemical path calculation method is the most highly accurate.

The alchemical path calculation method is also called as the alchemical free energy calculation or alchemical transformation, and is a method for calculating binding free energy using a thermodynamic cycle along a virtual (alchemical) path.

According to the alchemical path calculation method, binding free energy is calculated by calculating free energy of a process for restraining a distance or direction of a drug candidate molecule and free energy of a process of deleting interaction between the drug candidate molecule and the ambient environment thereof, and adding the calculated free energy values.

For example, the alchemical path calculation method is introduced in Adv Protein Chem Struct Biol. 2011; 85: 27-80.

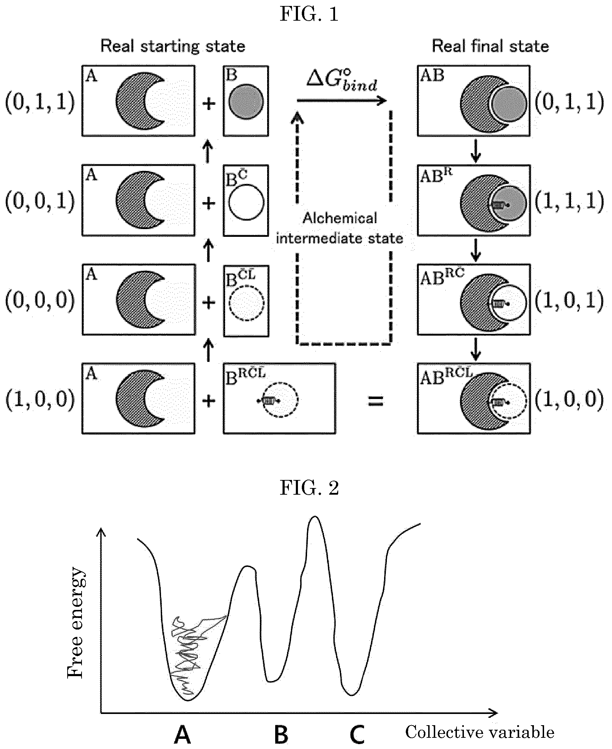

Examples of the alchemical path calculation method include a calculation method determined by FIG. 1 and the following equation.

$$\Delta G^{\circ}_{bind} = \{\Delta G(\mathrm{B} \to \mathrm{B}^{\bar{C}}) + \Delta G(\mathrm{B}^{\bar{C}} \to \mathrm{B}^{\bar{C}\bar{L}})\}$$

$$-\{\Delta G(\mathrm{AB}^{\mathrm{R}} \to \mathrm{AB}^{\mathrm{R}\bar{C}}) + \Delta G(\mathrm{AB}^{\mathrm{R}\bar{C}} \to \mathrm{AB}^{\mathrm{R}\bar{C}\bar{L}})\}$$

$$-\Delta G(\mathrm{B}^{\mathrm{R}\bar{C}\bar{L}} \to \mathrm{B}^{\bar{C}\bar{L}}) - \Delta G(\mathrm{AB} \to \mathrm{AB}^{\mathrm{R}})$$

In FIG. 1, the crescent-shaped object is a target molecule (A) and the circular object is a binding calculation target molecule (B). In the equation above and FIG. 1, C represents electrostatic interaction, L represents Van der Waals interaction, and R represent a spring restraint potential.

In the right side of the equation above, the first, second, third, fourth, and sixth items can be evaluated, for example, by the Bennett Acceptance Ratio (BAR) method and the EFP method.

The second term from the last in the right side of the formula above is often referred to as standard state correction [see, for example, non-patent literature (Michael S. Lee et. al., Calculation of Absolute Protein-Ligand Binding Affinity Using Path and Endpoint Approaches, Biophysical Journal, Volume 90, February 2006, 864-877)].

In order to perform the standard state correction, it is important to restrain a $\{\xi\}$ variable to a stable displacement in a calculation of binding free energy using the alchemical path calculation method. The variable is a variable for determining a direction of the drug candidate molecule relative to the target protein. Typically, harmonic potential is widely used in an external field where the restriction is performed.

In the case where simulation is performed according to the path illustrated in FIG. 1 in order to calculate binding free energy, a simulation with extremely weak restraint potential is performed in the state ($\varepsilon$, 1, 1) adjacent to (0, 1, 1) between the state (0, 1, 1) and the state (1, 1, 1).

In the case where the binding of the drug candidate molecule to the target protein has the free-energy surface as illustrated in FIG. 2 and each composite structure is sufficiently larger than room temperature fluctuations $k_BT$ (kB: Boltzmann's constant, T: temperature), the binding state remains to have the original composite state A, and the drug candidate molecule does not go outside a space volume $V_A$ set by the restraint potential in the thermodynamic cycle path of FIG. 1. Therefore, a standard state correction term can be correctly calculated according to the following formula.

$$\beta^{-1}\ln\left(\frac{V_A}{V^{\circ}}\right), \quad V^{\circ} = 1661 A^3$$

In the formula above, $\beta$ is $-1/k_BT$, where $k_B$ is the Boltzmann's constant and T is a temperature (K).

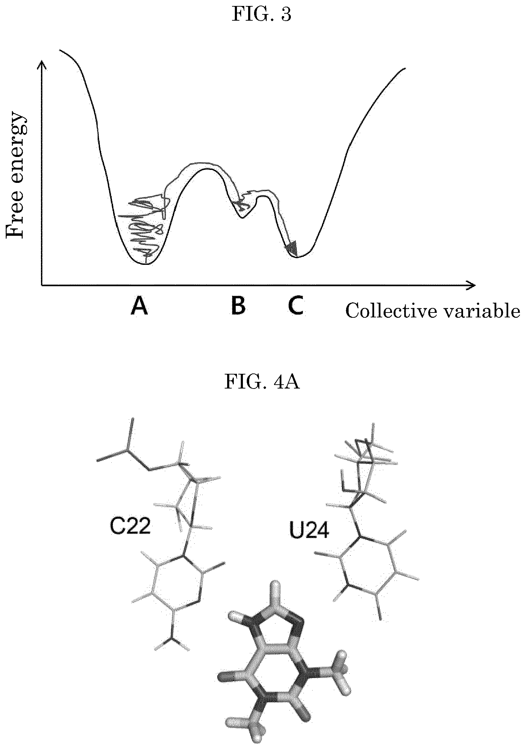

In the case where a free-energy surface of the binding of the drug candidate molecule to the target protein is the free-energy surface as illustrated in FIG. 3, in reality, the binding structure may go over the energy barrier due to room temperature fluctuations and the binding structure comes out from the state A to be in the state B then turned to be in the state C, even though the composite structure of the state A to which restraint potential is added should be sampled. In such a case, there is a problem that standard state correction cannot be performed because the drug candidate molecule goes outside the space set by the restraint potential.

The above-mentioned problem will be more specifically described.

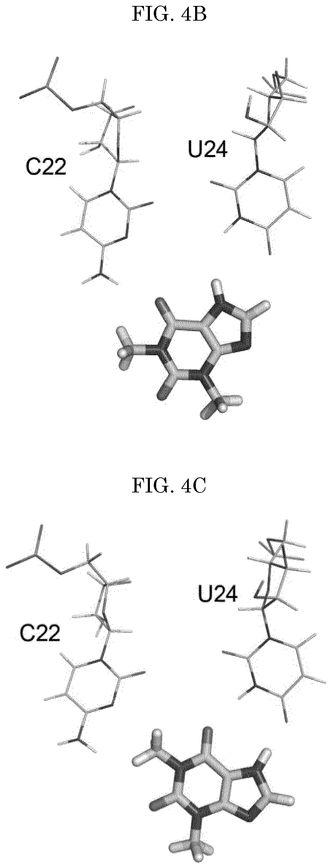

In the case where a ligand molecule is designed by building up from a low molecular weight molecule like a fragment, for example, a binding calculation target molecule often has a plurality of binding poses (stable binding structures) relative to a target molecule, as illustrated in FIGs. 4A, 4B, and 4C. FIGs. 4A, 4B, and 4C are views illustrating binding poses between RNA serving as a target molecule, and theophylline serving as a binding calculation target molecule. In FIGs. 4A, 4B, and 4C, C22 is cytosine and U24 is uracil.

In the above-described case, sampling is performed in a space where a valley of each potential energy is formed and binding free energy can be estimated, as long as valleys of potential energy representing binding poses are each separated with high energy barriers (typically, 6 $k_BT$ or greater, where $k_B$ is the Boltzmann's constant and T is a temperature), as illustrated in FIG. 2. In the case where the composite structures A, B, and C are present as in FIG. 2, specifically, the entire free energy can be calculated by the following formula with free energy at the valley of each potential energy being $\Delta G_i$ (i = A, B, C).

$$\Delta G^0$$

$$\cong -k_B T ln \left[ e^{-\frac{\Delta G_A}{k_B T}} + e^{-\frac{\Delta G_B}{k_B T}} + e^{-\frac{\Delta G_C}{k_B T}} \right]$$

$k_B$: Boltzmann's constant, $T$: temperature

In the case where energy barrier separating the valleys of potential energy is low, however, the binding structure may fall into the valley of another energy in the middle of the sampling, which changes a space volume of the sampling, and therefore it is impossible to perform quantitative estimation. In the case where the energy barrier separating the valleys of potential energy is low as illustrated in FIG. 3, for example, the composite structure is escaped from the state A, and moves from the state B to the state C.

The disclosed technology will be described by taking the case where harmonic potential having a spring constant $K_\xi$ is used as restraint potential as an example.

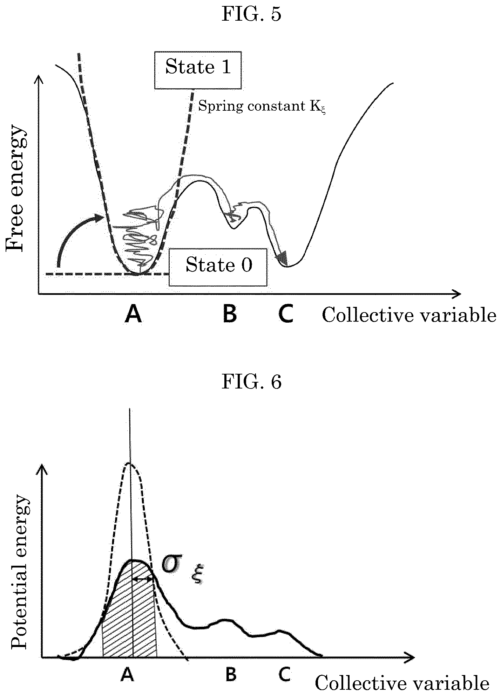

As illustrated in FIG. 5, harmonic potential having a spring constant $K_\xi$ as restraint potential is added to the valley A of potential energy. As a result the energy barrier with the valleys B and C of other potential energy becomes large and therefore transition of the binding structure does not occur. A change in free energy due to addition of the restraint potential is obtained by varying the spring constant $K_\xi$ with dividing into a few sections from 0 to $K_\xi$, and adding the change in free energy of each section together. According to the method as mentioned, there is still a possibility that the composite structure is escaped from the valley of the potential energy and moved into valleys of other potential energy in the region where $K_\xi$ is close to 0.

Note that, a density of the binding calculation target molecule is preferably constant during the simulation. In the case where a volume of a sampling region (searching space) changes along with the composite structure as described above, therefore, local binding energy cannot be determined.

Accordingly, the inventors of the disclosed technology have found that the data obtained by moving into valleys of other potential energy can be eliminated [in other words, a change in a volume of a sampling region (searching space) can be avoided] by selecting potential energy data present in a space created by the introduced restraint potential and using the selected data for a calculation of binding free energy, and therefore a quantitative calculation of binding free energy can be performed.

One example will be described with reference to FIG. 6.

Assuming that harmonic oscillation of the binding structure occurs in the variable space forming valleys of potential energy, the standard deviation $\sigma_\xi$ adjacent to the average value thereof at a temperature T (K) is represented by the following formula.

$$\langle \sigma_\xi \rangle = \frac{k_B T}{K_\xi}$$

In the formula above, T is a temperature (K), $K_\xi$ is a harmonic potential constant, and $k_B$ is the Boltzmann's constant.

Specifically, the space restrained to give a constant concentration is distributed as illustrated with the broken line of FIG. 6.

In the region where $K_\xi$ is close to 0, therefore, the data in the range of $\pm 3\sigma_\xi$ from the average value (the data in the shaded area of FIG. 6) is used for an analysis among the calculated data, and the other data is not used for the analysis. In the case where the space to which the maximum level of the introduced restraint potential $K_\xi$ is added causes the Gaussian distribution, 99.7% of the binding structure is present in the range of $\pm 3\sigma$. Therefore, the data in the range of $\pm 3\sigma_\xi$ is sufficient in view of calculation accuracy.

A quantitative calculation of binding free energy can be performed by analyzing without changing a volume of the sampling space as described above.

Specifically, the inventors of the disclosed technology have found the following insights and accomplished the disclosed technology based thereon.

When binding free energy between a binding calculation target molecule and a target molecule is calculated using an addition of restraint potential, the data present in the state A illustrated in FIGs. 2, 3, 5, and 6 is extracted from the entire simulation data obtained by searching a stable binding structure with adding restraint potential between the binding calculation target molecule and the target molecule to determine binding free energy, and therefore a calculation of binding free energy can be performed quantitatively even when there are a plurality of stable binding structures.

Moreover, the disclosed technology can be applied to, not only a combination of a binding calculation target molecule and a target molecule, but also any combination of a first substance and a second substance as long as the first substance and the second substance can form a composite.

<Potential energy determination step>

The potential energy determination step includes adding predetermined restraint potential between the first substance and the second substance to determine potential energy of a binding structure between the first substance and the second substance.

A combination of the first substance and the second substance is not particularly limited as long as the combination is a combination where the first substance and the second substance can form a composite, and may be appropriately selected depending on the intended purpose.

For example, the composite is formed with various interactions.

Examples of the first substance include a target molecule.

Examples of the second substance include a binding calculation target molecule.

The target molecule is not particularly limited and may be appropriately selected depending on the intended purpose. Examples of the target molecule include protein, ribonucleic acid (RNA), and deoxyribonucleic acid (DNA).

Examples of the binding calculation target molecule include a drug candidate molecule and a fragment for designing a drug candidate molecule.

For example, the fragment is used for fragment-based drug design (FBDD).

Moreover, examples of the first substance include antibodies and DNA, and examples of the second substance include pathogen, cancer cells, and stress-related substances. For example, the above-listed first substances are used for detecting the above-listed second substances. For example, the method for calculating binding free energy can be used for evaluating the capability of the first substance to detect the second substance.

The restraint potential is not particularly limited as long as the restraint potential is a potential for restraining a distance between the first substance and the second substance, and may be appropriately selected depending on the intended purpose. Examples of the restraint potential include harmonic potential.

The harmonic potential is a restraint potential for restraining with power proportional to a distance from a fixed point, and is also referred to as a restraint potential with a spring.

The addition of the harmonic potential is performed by adding harmonic potential with gradually increasing the harmonic potential from 0 to the maximum value. A change in the free energy caused by the addition of the harmonic potential can be determined, for example, by free energy perturbation (FEP), the Bennett acceptance ratio method (BAR), or thermo-dynamic integration (TI).

For example, the restraint potential is added between the first substance and the second substance using an anchor point of the first substance and an anchor point of the second substance.

For example, restraint potential added between an anchor point of the first substance and an anchor point of the second substance is determined in a manner that a size of fluctuations of the second substance is within a certain range.

For example, the distance restriction between the first substance and the second substance is performed in order to accurately consider a degree of freedom of translational motions of a molecule contributing the most to binding activity.

Accordingly, it is logical that a center of gravity of the second substance is set as an anchor point of the second substance. For example, a center of gravity of the second substance can be determined by the following equation.

$$\vec{x}_{\mathrm{com}} = \frac{\sum m_i \vec{x}_i}{\sum m_i}$$

In the formula above, m is a mass, and x is coordinates of an atom constituting the second substance.

Since a hydrogen atom is light, the hydrogen atom hardly affect a position of a center of gravity determined. Accordingly, a center of gravity of the second substance is preferably determined by excluding hydrogen atoms constituting the second substance because the calculation time can be shortened. Atoms excluding hydrogen atoms may be referred to as heavy atoms hereinafter.

<Potential energy selection step>

The potential energy selection step includes selecting potential energy used for a calculation of binding free energy from potential energy data present within a restraining space corresponding to a state where the maximum level of the predetermined restraint potential is added among the entire potential energy data determined in the potential energy determination step.

For example, the potential energy selection step includes selecting, as potential energy used for a calculation of binding free energy, potential energy data that is present within the restraining space corresponding to the state where the maximum level of the predetermined harmonic potential is added among the entire potential energy data determined in the potential energy determination step and is within a range determined using a standard deviation ($\sigma_\xi$) determined by Formula (1) below:

$$\sigma_\xi = \frac{k_{\mathrm{B}} T}{K_\xi} \qquad \text{Formula (1)}$$

In the formula above, T is a temperature (K), $K_\xi$ is a harmonic potential constant, and $k_B$ is the Boltzmann's constant.

The formula (1) expresses the relationship between the harmonic potential constant $K_\xi$ of harmonic oscillation and the standard deviation of the motions thereof.

It is assumed that the potential energy of the composite structure with room temperature fluctuations has a distribution close to the Gaussian distribution, when harmonic potential is added. Considering the viewpoint as mentioned, for example, the potential energy data within the range determined using the standard deviation ($\sigma_\xi$) gives high calculation accuracy of binding free energy because the potential energy data includes a large amount of potential energy data close to the peak of the state A of FIG. 6.

The range determined using the standard deviation ($\sigma_\xi$) is preferably a range of $\pm X\sigma$ (X is a number satisfying $1 \leq X \leq 4$) from the average value of the potential energy data within the restraining space, and is more preferably a range of $\pm 3\sigma$ from the average value of the potential energy data within the restraining space. The range of $\pm 3\sigma$ from the average value is appropriate as a range for sampling because the existence probability thereof is 99.7%.

<Binding free energy calculation step>

The binding free energy calculation step includes calculating binding free energy between the first substance and the second substance using the selected potential energy.

The method for calculating binding free energy is typically performed according to the alchemical path calculation method.

For example, the method for calculating binding free energy can be performed by means of a general computer system (e.g., various network servers, work stations, and personal computers) equipped with a central processing unit (CPU), random access memory (RAM), a hard disk, various peripherals, etc.

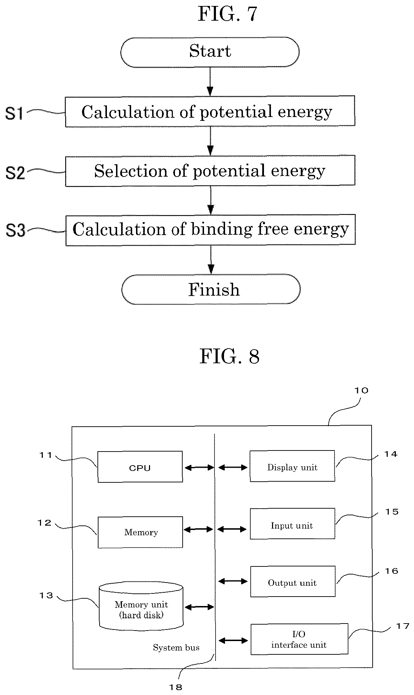

One example of the method for calculating binding free energy will be described with reference to a flowchart (FIG. 7).

First, predetermined restraint potential is added between a target molecule and a binding calculation target molecule to determine potential energy of a binding structure between the target molecule and the binding calculation target molecule (Step S1).

Next, among the entire potential energy data determined, potential energy data within the restraining space corresponding to the state where the maximum level of the predetermined restraint potential is added is selected (Step S2).

Next, binding free energy between the target molecule and the binding calculation target molecule is calculated from the selected potential energy data (Step S3).

(Program)

The disclosed program is a program for causing a computer to execute the disclosed method for calculating binding free energy.

The program can be created using any of various programing languages known in the art according to a configuration of a computer system for use, a type or version of an operation system for use.

The program may be recorded on storage media, such as an integral hard disk, and an external hard disk, or recorded on a storage medium, such as a compact disc read only memory (CD-ROM), a digital versatile disk read only memory (DVD-ROM), a magneto-optical (MO) disk, and a universal serial bus (USB) memory stick (USB flash drive). In the case where the program is recorded on a storage medium, such as a CD-ROM, a DVD-ROM, an MO disk, and an USB memory stick, the program can be used, as required, directly or by installing a hard disk via a storage medium reader equipped in a computer system. Moreover, the program may be recorded in an external memory region (e.g. another computer) accessible from the computer system via an information and communication network, and the program may be used, as required, by directly from the external memory region or installing into a hard disk from the external memory region via the information and communication network.

The program may be divided into predetermined processes and recorded on a plurality of non-transitory recording mediums.

(Computer-readable non-transitory recording medium)

The disclosed computer-readable non-transitory recording medium has stored therein the disclosed program.

The computer-readable non-transitory recording medium is not particularly limited and may be appropriately selected depending on the intended purpose. Examples of the computer-readable non-transitory recording medium include integral hard disks, external hard disks, CD-ROMs, DVD-ROMs, MO disks, and USB memory sticks.

The non-transitory recording medium may be a plurality of non-transitory recording mediums to which predetermined processes divided from the program are recorded.

(Device for calculating binding free energy)

The disclosed device for calculating binding free energy includes at least a potential energy calculating unit, a potential energy selecting unit, and a binding free energy calculating unit, and may further include other units according to the necessity.

The device for calculating binding free energy is a device for calculating binding free energy between a first substance and a second substance.

For example, the method for calculating binding free energy can be performed by the device for calculating binding free energy.

The potential energy calculating unit is configured to add predetermined restraint potential between the first substance and the second substance to determine potential energy of a binding structure between the first substance and the second substance.

The potential energy selecting unit is configured to select potential energy used for a calculation of binding free energy from potential energy data present within a restraining space corresponding to a state where the maximum level of the predetermined restraint potential is added among the entire potential energy data determined by the potential energy calculating unit.

The binding free energy calculating unit is configured to calculate binding free energy between the first substance and the second substance using the potential energy selected by the potential energy selecting unit.

A structural example of the disclosed device for calculating binding free energy is illustrated in FIG. 8.

For example, the device for calculating binding free energy 10 is composed by connecting CPU 11 (calculation unit), a memory 12, a memory unit 13, a display unit 14, an input unit 15, an output unit 16, and an I/O interface unit 17 via a system bus 18.

The central processing unit (CPU) 11 is configured to perform calculations (e.g., four arithmetic operations, and relational operations), and control of operations of hardware and software.

The memory 12 is a memory, such as a random access memory (RAM), and a read only memory (ROM). The RAM is configured to store an operating system (OS) and application programs read from the ROM and the memory unit 13, and function as a main memory and work area of the CPU 11.

The memory unit 13 is a device for storing various programs and data. For example, the memory unit 13 is a hard disk. In the memory unit 13, programs to be executed by the CPU 11, data for executing the programs, and an OS are stored.

The program is stored in the memory unit 13, loaded on the RAM (a main memory) of the memory 12, and executed by the CPU 11.

The display unit 14 is a display device. For example, the display unit is a display device, such as a CRT monitor, and a liquid crystal panel.

The input unit 15 is an input device for various types of data. Examples of the input unit include a key board, and a pointing device (e.g., a mouse).

The output unit 16 is an output device for various types of data. For example, the output unit is a printer.

The I/O interface unit 17 is an interface for connecting to various external devices. For example, the I/O interface unit enables input and output of data of CD-ROMs, DVD-ROMs, MO disks, and USB memory sticks.

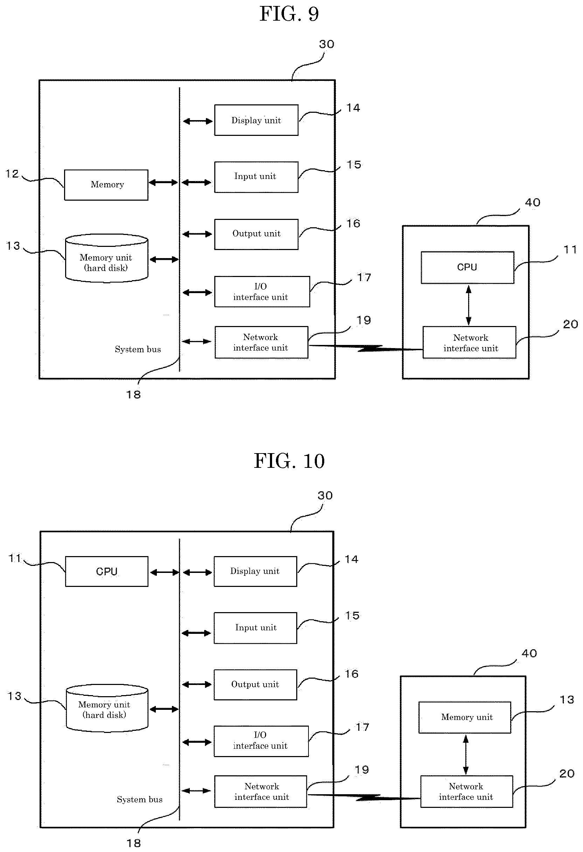

Another structural example of the disclosed device for calculating binding free energy is illustrated in FIG. 9.

The structural example of FIG. 9 is a structural example of a cloud-type calculation device, where CPU 11 is independent of a memory unit 13 etc. In the structural example, a computer 30 having stored therein the memory unit

EP 3 614 388 B1

13 and a computer 40 having stored therein the CPU 11 are coupled with each other via network interface units 19 and 20.

The network interface units 19 and 20 are hardware configured to communicate using Internet.

Another structural example of the disclosed device for calculating binding free energy is illustrated in FIG. 10.

The structural example of FIG. 10 is a structural example of a cloud-type calculation device, where a memory unit 13 is independent of CPU 11, etc. In the structural example, a computer 30 having stored therein the CPU 11 and a computer 40 having stored therein the memory unit 13 are coupled with each other via network interface units 19 and 20.

Examples

The disclosed technology will be described hereinafter, but Examples below shall not be construed as to limit the scope of the disclosed technology.

(Example 1)

A calculation of binding free energy of a system (PDB crystalline structure 1015) between a theophylline molecule and an RNA adaptor was performed.

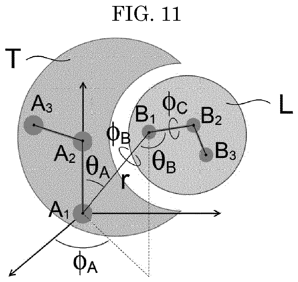

Potential energy was calculated by adding harmonic potential in a manner that variables presented in Table 1 were restrained in the positions illustrated in FIG. 11.

As presented in Table 1, 6 variables for determining a direction towards an RNA adaptor of a theophylline molecule were used as collective variables.

Table 1

| r | $\theta_A$ | $\theta_B$ | $\phi_A$ | $\phi_B$ | $\phi_C$ |
|---|---|---|---|---|---|
| 10.5 | 110.0 | 149.5 | - 62.1 | 118.7 | 164.2 |
| $K_r$ | $K_{\theta A}$ | $K_{\theta B}$ | $K_{\Phi A}$ | $K_{\phi B}$ | $K_{\phi C}$ |
| 2928.8 | 275.3 | 610.9 | 243.9 | 53.6 | 49.4 |

A unit for each coefficient in Table 1 is as follows.

r: Å
θ, Φ: (°)
$K_r$: kcal/mol/ Å$^2$
$K_\theta$, $K_\Phi$: kcal/mol/rad$^2$

In FIG. 11, the crescent-shaped object is the RNA adaptor that is the target molecule T, and the circular object is the theophylline molecule that is the binding calculation target molecule L.

Moreover, each of $A_1$, $A_2$, $A_3$, $B_1$, $B_2$, and $B_3$ is represented as follows using an atomic name and the number of PDB (1015).

$A_1$: C4'(689)
$A_2$: C3'(706)
$A_3$: O3'(712)
$B_1$: cc(1076)
$B_2$: cc(1080)
$B_3$: n(1073)

Moreover, $\varphi_A$ represents an angle between a plane formed with A1, A2, and A3, and a plane formed with A2, A1, and B1.

Among the obtained entire potential energy data, selected was potential energy that was the potential energy data within the restraining space corresponding to the state where the maximum level of the restraint potential was added, and was within the range of $\pm 3\sigma_\xi$ (with the proviso that $\sigma_\xi$ was the standard deviation determined by the formula below) from the average value of the potential energy data within the restraining space.

Then, binding free energy was calculated using the selected potential energy according to the alchemical path calculation method. As a result, the data presented in the columns of "$\pm 3\sigma$" of Table 2 below was obtained.

$$\sigma_\xi = \frac{k_B T}{K_\xi}$$

In the formula above, T is a temperature (K), $K_\xi$ is a harmonic potential constant, and $k_B$ is the Boltzmann's constant.

(Comparative Example 1)

Binding free energy was calculated in the same manner as in Example 1, except that the binding free energy was calculated using all of the obtained potential energy data. As a result, the data presented in the columns of "Original" of Table 2 below was obtained.

Table 2

| Interval time (ps) | Original | $\pm 3\sigma$ |
|---|---|---|
| 0-1000 | 1.522 (0.047) | 1.500 (0.049) |
| 4000-5000 | 1.887 (0.246) | 1.488 (0.030) |
| 9000-10000 | 2.979 (1.540) | 1.605 (0.052) |
| 14000-15000 | 4.078 (1.696) | |
| 19000-20000 | 4.292 (1.479) | |
| Unit: kcal/mol | | |

In Table 2, the numerical value within the bracket denotes a statistical error.
Table 2 demonstrates that the results of Example 1 had close values of the binding free energy calculated in the interval ranges, and the small statistical errors of the binding free energy values, compared to the results of Comparative Example 1. Specifically, it was confirmed that the disclosed method for calculating binding free energy could obtain the calculation results of excellent stability and high calculation accuracy compared to the conventional method of Comparative Example 1.

**Claims**

1. A method for calculating binding free energy between a first substance and a second substance, the method comprising:

   increasing (S1), in a plurality of steps, a distance restraint potential between the first substance and the second substance from 0 to a maximum level, thereby obtaining multiple potential energy data by calculating potential energy data of a binding structure between the first substance and the second substance at each of the plurality of steps;
   selecting (S2), from the multiple potential energy data, first potential energy data within a restrained space, the restrained space corresponding to a state where the distance restraint potential is at the maximum level; and
   calculating (S3) the binding free energy between the first substance and the second substance using the first potential energy data selected,
   wherein the method is a method for calculating the binding free energy between the first substance and the second substance using a computer.

2. The method according to claim 1,
   wherein the first substance is a target molecule and the second substance is a binding calculation target molecule.

3. The method according to claim 1 or 2,
   wherein the distance restraint potential is a harmonic potential.

4. The method according to any of claims 1 to 3,
   wherein the first potential energy data is within a range determined using a standard deviation, $\sigma_\xi$, determined by

Formula (1) below,

$$\sigma_\xi = \frac{k_\mathrm{B}T}{K_\xi} \quad \text{Formula (1)}$$

where T is a temperature in kelvin, $K_\xi$ is a harmonic potential constant, and $k_\mathrm{B}$ is the Boltzmann's constant.

5. The method according to claim 4,
wherein the range determined using the standard deviation, $\sigma_\xi$, is a range that is $\pm X\sigma$, where X is a number satisfying $1 \le X \le 4$, from an average value of the potential energy data within the restrained space.

6. The method according to claim 4 or 5,
wherein the range determined using the standard deviation, $\sigma_\xi$, is a range that is $\pm 3\sigma$ from an average value of the potential energy data within the restrained space.

7. The method according to any one of claims 1 to 6,
wherein the method is performed according to an alchemical path calculation method.

8. A program for causing a computer to execute a method for calculating binding free energy between a first substance and a second substance, the method comprising:

increasing (S1), in a plurality of steps, a distance restraint potential between the first substance and the second substance from 0 to a maximum level, thereby obtaining multiple potential energy data by calculating potential energy data of a binding structure between the first substance and the second substance;
selecting (S2), from the multiple potential energy data, first potential energy data within a restrained space, the restrained space corresponding to a state where the distance restraint potential is at the maximum level; and
calculating (S3) the binding free energy between the first substance and the second substance using the first potential energy data selected.

9. The program according to claim 8,
wherein the first substance is a target molecule and the second substance is a binding calculation target molecule.

10. The program according to claim 8 or 9, wherein the distance restraint potential is a harmonic potential.

11. The program according to claim 10, wherein the first potential energy data is within a range determined using a standard deviation, $\sigma_\xi$, determined by Formula (1) below,

$$\sigma_\xi = \frac{k_\mathrm{B}T}{K_\xi} \quad \text{Formula (1)}$$

where T is a temperature in kelvin, $K_\xi$ is a harmonic potential constant, and $k_\mathrm{B}$ is the Boltzmann's constant.

12. A device for calculating binding free energy between a first substance and a second substance, the device comprising:

a potential energy calculation unit configured to increase, in a plurality of steps, a distance restraint potential between the first substance and the second substance from 0 to a maximum level, thereby obtaining multiple potential energy data by calculating potential energy data of a binding structure between the first substance and the second substance at each of the plurality of steps;
a potential energy selecting unit configured to select, from the multiple potential energy data, first potential energy data within a restrained space, the restrained space corresponding to a state where the distance restraint potential is at the maximum level; and
a binding free energy calculation unit configured to calculate the binding free energy between the first substance and the second substance using the first potential energy data selected by the potential energy selecting unit.

**13.** The device according to claim 12,
wherein the first substance is a target molecule and the second substance is a binding calculation target molecule.

**14.** The device according to claim 12 or 13, wherein the distance restraint potential is a harmonic potential.

**15.** The device according to claim 14, wherein the first potential energy data is within a range determined using a standard deviation, $\sigma_\xi$, determined by Formula (1) below,

$$\sigma_\xi \ = \frac{k_\mathrm{B} T}{K_\xi} \qquad \text{Formula (1)}$$

where T is a temperature in kelvin, $K_\xi$ is a harmonic potential constant, and $k_\mathrm{B}$ is the Boltzmann's constant.

**Patentansprüche**

**1.** Verfahren zur Berechnung der freien Bindungsenergie zwischen einer ersten Substanz und einer zweiten Substanz, wobei das Verfahren Folgendes umfasst:

Erhöhen (S1) eines Abstandsbeschränkungspotenzials zwischen der ersten Substanz und der zweiten Substanz von 0 auf ein maximales Niveau in einer Vielzahl von Schritten, wodurch durch Berechnen von Daten zu potenzieller Energie einer Bindungsstruktur zwischen der ersten Substanz und der zweiten Substanz bei jedem der Vielzahl von Schritten mehrere Daten zu potenzieller Energie erhalten werden;
Auswählen (S2) erster Daten zu potenzieller Energie innerhalb eines beschränkten Raums aus den mehreren Daten zu potenzieller Energie, wobei der beschränkte Raum einem Zustand entspricht, in dem das Abstandsbeschränkungspotenzial auf dem maximalen Niveau ist; und
Berechnen (S3) der bindungsfreien Energie zwischen der ersten Substanz und der zweiten Substanz unter Verwendung der ausgewählten ersten Daten zu potenzieller Energie, wobei das Verfahren ein Verfahren zur Berechnung der bindungsfreien Energie zwischen der ersten Substanz und der zweiten Substanz unter Verwendung eines Computers ist.

**2.** Verfahren nach Anspruch 1,
wobei die erste Substanz ein Zielmolekül ist und die zweite Substanz ein Bindungsberechnungs-Zielmolekül ist.

**3.** Verfahren nach Anspruch 1 oder 2,
wobei das Abstandsbeschränkungspotenzial ein harmonisches Potenzial ist.

**4.** Verfahren nach einem der Ansprüche 1 bis 3,
wobei die ersten Daten zu potenzieller Energie innerhalb eines Bereichs liegen, der unter Verwendung einer durch die nachstehende Formel (1) bestimmten Standardabweichung, $\sigma_\xi$, bestimmt wird,

$$\sigma_\xi \ = \frac{k_\mathrm{B} T}{K_\xi} \qquad \text{Formel (1)}$$

wobei T eine Temperatur in Kelvin ist, $K_\xi$ eine harmonische Potenzialkonstante ist und $k_\mathrm{B}$ die Boltzmann-Konstante ist.

**5.** Verfahren nach Anspruch 4,
wobei der unter Verwendung der Standardabweichung, $\sigma_\xi$, bestimmte Bereich ein $\pm X\sigma$ betragender Bereich um einen Durchschnittswert der Daten zu potenzieller Energie innerhalb des beschränkten Raums ist, wobei X eine Zahl ist, die $1 \leq X \leq 4$ erfüllt.

**6.** Verfahren nach Anspruch 4 oder 5,

wobei der unter Verwendung der Standardabweichung, $\sigma_\xi$, bestimmte Bereich ein $\pm 3\sigma$ betragender Bereich um einen Durchschnittswert der Daten zu potenzieller Energie innerhalb des beschränkten Raums ist.

7. Verfahren nach einem der Ansprüche 1 bis 6,
   wobei das Verfahren gemäß einem alchemistischen Pfadberechnungsverfahren durchgeführt wird.

8. Programm zum Veranlassen eines Computers, ein Verfahren zur Berechnung von freier Bindungsenergie zwischen einer ersten Substanz und einer zweiten Substanz auszuführen, wobei das Verfahren Folgendes umfasst:

   Erhöhen (S1) eines Abstandsbeschränkungspotenzials zwischen der ersten Substanz und der zweiten Substanz von 0 auf ein maximales Niveau in einer Vielzahl von Schritten, wodurch durch Berechnen von Daten zu potenzieller Energie einer Bindungsstruktur zwischen der ersten Substanz und der zweiten Substanz mehrere Daten zu potenzieller Energie erhalten werden;
   Auswählen (S2) erster Daten zu potenzieller Energie innerhalb eines beschränkten Raums aus den mehreren Daten zu potenzieller Energie, wobei der beschränkte Raum einem Zustand entspricht, in dem das Abstandsbeschränkungspotenzial auf dem maximalen Niveau ist; und
   Berechnen (S3) der bindungsfreien Energie zwischen der ersten Substanz und der zweiten Substanz unter Verwendung der ausgewählten ersten Daten zu potenzieller Energie.

9. Programm nach Anspruch 8,
   wobei die erste Substanz ein Zielmolekül ist und die zweite Substanz ein Bindungsberechnungs-Zielmolekül ist.

10. Programm nach Anspruch 8 oder 9,
    wobei das Abstandsbeschränkungspotenzial ein harmonisches Potenzial ist.

11. Programm nach Anspruch 10,
    wobei die ersten Daten zu potenzieller Energie innerhalb eines Bereichs liegen, der unter Verwendung einer durch die nachstehende Formel (1) bestimmten Standardabweichung, $\sigma_\xi$, bestimmt wird,

$$\sigma_\xi = \frac{k_\mathrm{B} T}{K_\xi}$$

Formel (1)

wobei T eine Temperatur in Kelvin ist, $K_\xi$ eine harmonische Potenzialkonstante ist und $k_\mathrm{B}$ die Boltzmann-Konstante ist.

12. Vorrichtung zur Berechnung der freien Bindungsenergie zwischen einer ersten Substanz und einer zweiten Substanz, wobei die Vorrichtung Folgendes umfasst:

    eine Einheit zur Berechnung von potenzieller Energie, die zum Erhöhen eines Abstandsbeschränkungspotenzials zwischen der ersten Substanz und der zweiten Substanz von 0 auf ein maximales Niveau in einer Vielzahl von Schritten konfiguriert ist, wodurch durch Berechnen von Daten zu potenzieller Energie einer Bindungsstruktur zwischen der ersten Substanz und der zweiten Substanz bei jedem der Vielzahl von Schritten mehrere Daten zu potenzieller Energie erhalten werden;
    eine Einheit zum Auswählen von potenzieller Energie, die zum Auswählen erster Daten zu potenzieller Energie innerhalb eines beschränkten Raums aus den mehreren Daten zu potenzieller Energie konfiguriert ist, wobei der beschränkte Raum einem Zustand entspricht, in dem das Abstandsbeschränkungspotenzial auf dem maximalen Niveau ist; und
    eine Einheit zur Berechnung von freier Bindungsenergie, die zum Berechnen der bindungsfreien Energie zwischen der ersten Substanz und der zweiten Substanz unter Verwendung der von der Einheit zum Auswählen von potenzieller Energie ausgewählten ersten Daten zu potenzieller Energie konfiguriert ist.

13. Vorrichtung nach Anspruch 12,
    wobei die erste Substanz ein Zielmolekül ist und die zweite Substanz ein Bindungsberechnungs-Zielmolekül ist.

14. Vorrichtung nach Anspruch 12 oder 13,

wobei das Abstandsbeschränkungspotenzial ein harmonisches Potenzial ist.

**15.** Vorrichtung nach Anspruch 14,
wobei die ersten Daten zu potenzieller Energie innerhalb eines Bereichs liegen, der unter Verwendung einer durch die nachstehende Formel (1) bestimmten Standardabweichung, $\sigma_\xi$, bestimmt wird,

$$\sigma_\xi \;=\; \frac{k_{\mathrm{B}}T}{K_\xi}$$

Formel (1)

wobei T eine Temperatur in Kelvin ist, $K_\xi$ eine harmonische Potenzialkonstante ist und $k_{\mathrm{B}}$ die Boltzmann-Konstante ist.

## Revendications

**1.** Procédé de calcul de l'énergie sans liaison entre une première substance et une seconde substance, le procédé comprenant :

l'augmentation (S1), dans une pluralité d'étapes, d'un potentiel de restriction de distance entre la première substance et la seconde substance de 0 à un niveau maximal, obtenant ainsi de multiples données d'énergie potentielle en calculant des données d'énergie potentielle d'une structure de liaison entre la première substance et la seconde substance à chacune de la pluralité d'étapes ;
la sélection (S2), à partir des multiples données d'énergie potentielle, des premières données d'énergie potentielle dans un espace restreint, l'espace restreint correspondant à un état où le potentiel de restriction de distance est au niveau maximal ; et
le calcul (S3) de l'énergie sans liaison entre la première substance et la seconde substance à l'aide des premières données d'énergie potentielle sélectionnées,
dans lequel le procédé est un procédé de calcul de l'énergie sans liaison entre la première substance et la seconde substance à l'aide d'un ordinateur.

**2.** Procédé selon la revendication 1,
dans lequel la première substance est une molécule cible et la seconde substance est une molécule cible de calcul de liaison.

**3.** Procédé selon la revendication 1 ou 2,
dans lequel le potentiel de restriction de distance est un potentiel harmonique.

**4.** Procédé selon l'une quelconque des revendications 1 à 3, dans lequel les premières données d'énergie potentielle se situent dans une plage déterminée à l'aide d'un écart type, $\sigma_\xi$, déterminé par la Formule (1) ci-dessous,

$$\sigma_\xi \;=\; \frac{k_{\mathrm{B}}T}{K_\xi}$$

Formule (1)

où T est une température en kelvin, $K_\xi$ est une constante de potentiel harmonique et $k_{\mathrm{B}}$ est la constante de Boltzmann.

**5.** Procédé selon la revendication 4,
dans lequel la plage déterminée à l'aide de l'écart type, $\sigma_\xi$, est une plage qui est $\pm X\sigma$, où X est un nombre satisfaisant $1 \le X \le 4$, à partir d'une valeur moyenne des données d'énergie potentielle dans l'espace restreint.

**6.** Procédé selon la revendication 4 ou 5,
dans lequel la plage déterminée à l'aide de l'écart type, $\sigma_\xi$, est une plage qui est $\pm 3\sigma$ à partir d'une valeur moyenne des données d'énergie potentielle dans l'espace restreint.

7. Procédé selon l'une quelconque des revendications 1 à 6, dans lequel le procédé est réalisé selon un procédé de calcul de chemin alchimique.

8. Programme permettant à un ordinateur d'exécuter un procédé de calcul de l'énergie sans liaison entre une première substance et une seconde substance, le procédé comprenant :

l'augmentation (S1), dans une pluralité d'étapes, d'un potentiel de restriction de distance entre la première substance et la seconde substance de 0 à un niveau maximal, obtenant ainsi de multiples données d'énergie potentielle en calculant des données d'énergie potentielle d'une structure de liaison entre la première substance et la seconde substance ;
la sélection (S2), à partir des multiples données d'énergie potentielle, des premières données d'énergie potentielle dans un espace restreint, l'espace restreint correspondant à un état où le potentiel de restriction de distance est au niveau maximal ; et
le calcul (S3) de l'énergie sans liaison entre la première substance et la seconde substance à l'aide des premières données d'énergie potentielle sélectionnées.

9. Programme selon la revendication 8,
dans lequel la première substance est une molécule cible et la seconde substance est une molécule cible de calcul de liaison.

10. Programme selon la revendication 8 ou 9,
dans lequel le potentiel de restriction de distance est un potentiel harmonique.

11. Programme selon la revendication 10,
dans lequel les premières données d'énergie potentielle se situent dans une plage déterminée à l'aide d'un écart type, $\sigma_\xi$, déterminé par la Formule (1) ci-dessous,

$$\sigma_\xi \;=\; \frac{k_{\mathrm{B}}T}{K_\xi}$$

$$\texttt{Formule (1)}$$

où T est une température en kelvin, $K_\xi$ est une constante de potentiel harmonique et $k_B$ est la constante de Boltzmann.

12. Dispositif de calcul de l'énergie sans liaison entre une première substance et une seconde substance, le dispositif comprenant :

une unité de calcul de l'énergie potentielle configurée pour augmenter, dans une pluralité d'étapes, un potentiel de restriction de distance entre la première substance et la seconde substance de 0 à un niveau maximal, obtenant ainsi de multiples données d'énergie potentielle en calculant des données d'énergie potentielle d'une structure de liaison entre la première substance et la seconde substance à chacune de la pluralité d'étapes ;
une unité de sélection de l'énergie potentielle configurée pour sélectionner, à partir des multiples données d'énergie potentielle, des premières données d'énergie potentielle dans un espace restreint, l'espace restreint correspondant à un état où le potentiel de restriction de distance est au niveau maximal ; et
une unité de calcul de l'énergie sans liaison configurée pour calculer l'énergie sans liaison entre la première substance et la seconde substance à l'aide des premières données d'énergie potentielle sélectionnées par l'unité de sélection de l'énergie potentielle.

13. Dispositif selon la revendication 12,
dans lequel la première substance est une molécule cible et la seconde substance est une molécule cible de calcul de liaison.

14. Dispositif selon la revendication 12 ou 13,
dans lequel le potentiel de restriction de distance est un potentiel harmonique.

15. Dispositif selon la revendication 14,
dans lequel les premières données d'énergie potentielle se situent dans une plage déterminée à l'aide d'un écart

type, $\sigma_\xi$, déterminé par la Formule (1) ci-dessous,

$$\sigma_\xi = \frac{k_B T}{K_\xi}$$ Formule (1)

où T est une température en kelvin, $K_\xi$ est une constante de potentiel harmonique et $k_B$ est la constante de Boltzmann.

FIG. 1

FIG. 2

FIG. 3

FIG. 4A

FIG. 4B

FIG. 4C

FIG. 5

FIG. 6

FIG. 7

```
                    ┌──────────────────┐
                    │      Start       │
                    └──────────────────┘
                             │
                             ▼
  S1 ──┤ ┌────────────────────────────────────────┐
       │ │     Calculation of potential energy     │
       │ └────────────────────────────────────────┘
                             │
                             ▼
  S2 ──┤ ┌────────────────────────────────────────┐
       │ │      Selection of potential energy      │
       │ └────────────────────────────────────────┘
                             │
                             ▼
  S3 ──┤ ┌────────────────────────────────────────┐
       │ │   Calculation of binding free energy    │
       │ └────────────────────────────────────────┘
                             │
                             ▼
                    ┌──────────────────┐
                    │      Finish      │
                    └──────────────────┘
```

FIG. 8

## FIG. 9

## FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 3327604 A1
- JP 2017091180 A
- WO 2017199279 A1

### Non-patent literature cited in the description

- **JOHN D. CHODERA.** Alchemical free energy methods for drug discovery: Progress and challenges. *Curr Opin Struct Biol.,* April 2011, vol. 21 (2), 150-160
- *Adv Protein Chem Struct Biol.,* 2011, vol. 85, 27-80
- **MICHAEL S. LEE.** Calculation of Absolute Protein-Ligand Binding Affinity Using Path and Endpoint Approaches. *Biophysical Journal,* February 2006, vol. 90, 864-877